# EUROPEAN PATENT SPECIFICATION

(11) **EP 1 523 287 B1**
(45) Date of publication and mention of the grant of the patent: **12.01.2011**
(21) Application number: 03760228.1
(22) Date of filing: 29.05.2003
(51) Int. Cl.: A61F 2/02, C08F 290/06

(54) **MATERIALS WITH BOTH BIOADHESIVE AND BIODEGRADABLE COMPONENTS**
MATERIALIEN MIT BIOADHÄSIVEN UND BIOLOGISCH ABBAUBAREN KOMPONENTEN
MATERIAUX COMPRENANT A LA FOIS DES ELEMENTS BIOADHESIFS ET DES ELEMENTS BIODEGRADABLES

(30) Priority: 14.06.2002 US 389022 P; 22.11.2002 US 302484
(43) Date of publication of application: 20.04.2005
(73) Proprietor: KIMBERLY-CLARK WORLDWIDE, INC., Neenah, WI 54956 (US)
(72) Inventor: HUANG, Yanbin, Roswell, GA 30076 (US); KIM, Jaeho, Roswell, GA 30075 (US)
(74) Representative: Beacham, Annabel Rose
(86) International application number: PCT/US2003/016919
(87) International publication number: WO 2003/105661

(56) References cited:
- WO-A-01/68157
- WO-A-97/47670
- US-A- 3 882 057
- US-A- 5 474 768

## Description

### BACKGROUND OF THE INVENTION

The present invention concerns bioadhesive polymers incorporated with biodegradable components for use in helping to maintain a proper pH in body cavities like the vagina, and for the delivery of treating agents.

As used herein, a bioadhesive is a material that adheres to biological surfaces such as mucus membrane or skin tissue. When being applied as drug delivery systems, they have the following desirable features: (1) localization in specific regions, (2) optimum contact with the biological surface, and (3) prolonged residence time. More information about types and applications of bioadhesive polymers can be found in, for example, "Surface, interfacial and molecular aspects of polymer bioadhesion on soft tissues" by NA Peppas and PA Buri, Journal of Controlled Release, 1985, 2, 257-275; "Binding of acrylic polymers to mucin/epithelial surfaces: structure-property relationships" by JM Gu, JR Robinson and SH Leung, Critical Reviews of Therapeutic Drug Carrier Systems, 1988, 5, 21-67; and "Bioadhesives for optimization of drug delivery", by NA Peppas and JR Robinson, Journal of Drug Targeting, 1995, 3, 183-184. Polycarboxylic acids are among the most widely used bioadhesive polymers, and their biomedical applications are documented in, for example, US Patent 5,225,196.

Biodegradable polymers have numerous applications in the biomedical field such as wound closures, body implants, tissue engineering, and in drug delivery. Biodegradable polymers, as used herein, are polymers which are degradable in a physiologically relevant environment, either by hydrolysis, by enzymatic reactions or by other mechanisms, to produce biocompatible or non-toxic by products. This is further discussed in "Biodegradable Polymeric Biomaterials: An Overview", by Chih-Chang Chu, in The Biomedical Engineering Handbook, edited by JD Bronzino, CRC Press, 1995 and "Biodegradable Polymers and Their Degradation Mechanisms" by Yichun Sun et al., in American Pharmaceutical Reviews, 2001, 4(3), 9-18. More information on the making of synthetic biodegradable polymers can be found in "Chemical Synthesis of Biodegradable Polymers" by Masahiko Okada, in Progress in Polymer Science, 2002, 27, 87-133.

The pH of the vagina of a healthy female is in the acidic range (pH 3.5-4.5) and is generated and maintained by the production of lactic acid by *Lactobacilli,* which forms a major component of the healthy vaginal flora. Together with other factors, this acidic pH is widely recognized as preventing overgrowth of undesirable endogenous microbes (Candida, harmful anaerobes, and other bacteria that may cause urinary tract infections) and encourages the continued dominance of *Lactobacilli* which, in addition to mild acidity, provides other protective mechanisms such as production of hydrogen peroxide.

It is also known that sperm are inactivated by the mild acidity of the vagina of a healthy female, and acid substances have been used as home made vaginal contraceptives for centuries. More recently it has been recognized that many sexually transmitted disease pathogens such as *Neisseria* gonorrhea, *Treponema pallidum, Haemophilus ducreyi,* and most or all enveloped sexually transmitted viruses including herpes simplex virus, cytomegalovirus, and human immunodeficiency virus, are also inhibited or inactivated by mild acidic pH.

Maintaining an acidic vaginal environment during menstruation would also have benefits. During menstruation the protective vaginal acidity is temporarily lost due to the rate at which neutral and strongly buffering menstrual fluids enter the vagina. Consequently, acid inhibition of deleterious vaginal flora such as *Staphylococcus aureus, Candida albicans,* harmful anaerobes, and other bacteria that may cause urinary tract infections is lost for a period of 4-7 days each menstrual cycle.

Elevated pH also allows certain strains of *Staphylococcus aureus* to produce toxic shock toxin I, whereas production of this toxin has been shown to be inhibited at a pH of less than 5 by Schlievert et al. in Journal of Infectious Disease, 11983, 47, 236-242. The loss of protective acidity may therefore result in serious consequences. Reestablishing vaginal acidity may be therapeutic in reversing established vaginitis (by *Candida* and *Trichomonas* vaginitis) and the non-inflammatory condition termed bacterial vaginosis, characterized by an elevated vaginal pH due to reduction in *Lactobacilli* populations and increase in other vaginal anaerobic bacteria. Acid pH also inhibits the harmful anaerobes whose overgrowth is associated with the malodorous discharge of bacterial vaginosis.

Attempts have been made to treat these conditions with acidifying gels, with some success as discussed, for example by Holst et al., in J. Infectious Disease, 1990, 22, 625-626. The effectiveness of these products is limited, however, by their limited buffering capacity, and the fact that they may drip out of the vagina, and because the acidifying agent may be absorbed across the vaginal mucosa. Results suggest that a method that provides greater acidic buffer capacity, and that used a buffer that could be fully retained in the vagina would improve the therapeutic performance of this method for treating common vaginal infections.

Despite the recognition that acidic buffering of the vagina could protect against many sexually transmitted diseases and other deleterious vaginal conditions, an appropriate vaginal buffering strategy has not previously been devised. Many buffers that might be employed would be ineffective due to their toxicity. The acidifying power required is large and many types of acidic buffers would be excessively hypertonic and/or caustic if employed in sufficient dose. Second, many possible buffers are small molecules that rapidly leave the vagina by diffusing through the vaginal mucosa, thus limiting the duration of protection they can provide.

On the other hand, biodegradable polymers, particularly degradable polyesters such as poly(lactic acid), have been known to be degraded in an aqueous environment and to release smaller molecular acids like lactic acid, which naturally exists in the healthy vagina. The numerous biomedical applications of these polymers focus on their degradability, but not the use of the acidic degradation products.

There remains a need for materials capable of buffering the pH in body cavities like the vagina that have more longevity than existing materials.

### SUMMARY OF THE INVENTION

The current invention concerns bioadhesive polymers incorporated with biodegradable components, as defined in claim 1. The bioadhesive components used are polycarboxylic acids, particularly poly(acrylic acid), poly(methacrylic acid), and their copolymers, while the biodegradable components are biodegradable polyesters whose degradation products are low molecular weight acids, selected from poly(lactic acid), poly(lactic-co-glycolic acid), PEG-PLGA copolymers and PVA-PLGA copolymers.

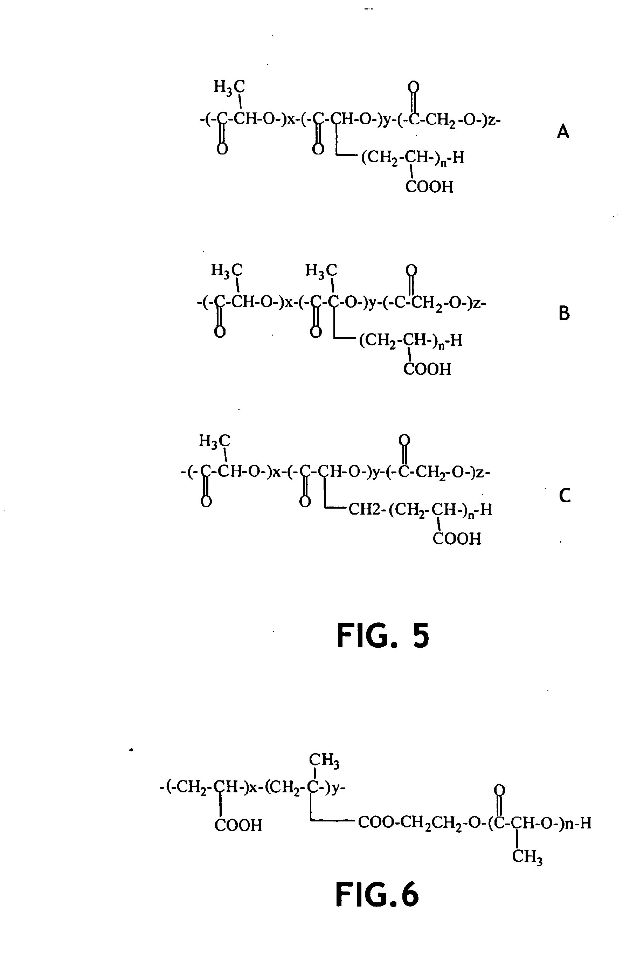

The two polymer components may be covalently bound to each other forming graft, block or random copolymers. Such polymers are poly(acrylic acid)-graft-poly(lactic acid) (PAA-g-PLA) and poly(lactic-co-glycolic acid)-g-poly(acrylic acid) (PLGA-g-PAA), the making of which are described in the examples below. Possible structures for PLGA-g-PAA are given in Figure 5 A, B and C. The structure of PAA-g-PLA is given in Figure 6. The two components may also be incorporated into one material by forming a non-covalent interpolymer complex via hydrogen bonding. These polymers are the hydrogen bonding interpolymer complex formed between poly(methacrylic acid) and poly(lactic acid)-b-poly(ethylene glycol)-b-poly(lactic acid) tri-block copolymers, or more generally, complexes formed between polycarboxylic acids and PEG-PLGA copolymers or PVA-PLGA copolymers.

The new materials may be used without including a treatment agent, to provide a lasting acidic pH environment. In this embodiment, the bioadhesive property enables the material to adhere strongly to biological surfaces, while the degradation of the biodegradable polyesters releases non-toxic low molecular weight acids. An acidic pH is very important to maintain or to establish a healthy body cavity environment such as, for example, in the vaginal cavity.

The new materials may also be used as a treatment agent delivery system and the treating agent may be an agent, for example, for treating menstruation disorders and infections, agent for treating cardiovascular conditions, agents for treating internal conditions, agents for treating mental health conditions, anti-inflammatory agents, chemotherapeutic agents, cardiac tonics, expectorants, oral antiseptics, enzymes, birth control agents, ophthalmic treating agents and combinations thereof.

These new materials can be either used without loading active agents as bioadhesive systems slowly releasing low molecular weight acids, or loaded with active agents as bioadhesive drug delivery systems.

### BRIEF DESCRIPTION OF THE DRAWINGS

Figures 1 and 2 are 1H-and 13C-NMR graphs of the reaction product of Example 1.
Figures 3 and 4 are 1H-and 13C-NMR graphs of the reaction product of Example 2.
Figure 5 is a depiction of three possible structures for PLGA-g-PAA (Figure 5 A, B and C).
Figure 6 is a depiction of the structure of PAA-g-PLA.

### DETAILED DESCRIPTION OF THE INVENTION

As briefly described above, the current invention provides novel materials combining bioadhesive components and biodegradable components. Examples of their applications include systems maintaining acidic body cavity environments and novel drug delivery systems.

The factors that affect the degradation rate of the polymers are: (1) chemical properties such as the structure of monomers and the composition of monomers, (2) physical properties such as hydrophilicity and crystalline degrees, (3) the molecular weight of the polymers, (4) geometric factors of the product such as size, shape and surface area, and (5) additives and environmental factors, such as pH and ionic strength.

Most synthetic biodegradable polymers such as poly(lactic-co-glycolic acid) (PLGA) are hydrophobic. The combination of these hydrophobic polymers with hydrophilic bioadhesive polymers provides control for the degradation rate and processability along with the bioadhesive property of the resulting polymer.

One aspect of the current invention is, therefore, to provide bioadhesive polymers incorporated with biodegradable components, and to use these new materials to maintain or establish a lasting acidic pH environment in certain body cavities such as the vagina. The bioadhesive property enables a relatively long residence time for the whole system after it is applied, while the degradation of the biodegradable components releases the physiologically-acceptable acids.

The bioadhesive property of the polymer enables a relatively long residence time for the whole system after it is applied, while the degradation of the biodegradable components releases the physiologically-acceptable acids. The release of the physiologically-acceptable acids occurs as PLGA is hydrolyzed in the aqueous environment of the body cavity. The degradation of the polymer results in the release of small molecular weight lactic acids and glycolic acids which have a pKa of 3.08 and 3.83 respectively. The degradation kinetics have been studied previously. Achim Gopferich's article "Mechanisms of Polymer Degradation and Erosion" (Biomaterials 1996, vol. 17, no. 2, p. 103- 114, published by Elsevier Science Limited) discusses the release of oligomers and monomers from the base polymer by erosion and diffusion. Likewise the efficacy of these polymers is discussed in EP 0 797 457 B1. The '457 patent discusses prior attempts to deliver these polymers by inserting lactic acid producing bacteria into the body cavity via, for example, a tampon. Other methods of delivering beneficial acidic polymers are also discussed because lactic acid is known to have an inhibiting effect on various pathogenic microorganisms by lowering the pH in, particularly, the urogenital environment.

The bioadhesive should contain units of carboxyl functionality and these should be present in an amount of about 80 percent of the repeating units of the bioadhesive. Exemplary monomers that provide these repeating units are monoethylenically unsaturated and include acrylic acid, methacrylic acid, fumaric acid, maleic acid, maleic anhydride which may be hydrolyzed into its acid form during or after polymerization, itaconic acid, crotonic acid, and so forth. Each of these acids may be used alone or in combination with other such acids or with one or more pharmaceutically or cosmetically acceptable salts of those acids. Acrylic acid is a particularly desirable monomer for providing the repeating units of the bioadhesive polymer.

The materials of the invention can be used alone or together with other materials and agents, as delivery vehicles in the form of solutions, creams, lotions, gels and other vagina inserts such as tampons, sponges, and rings. The materials of this invention may be added to these materials in an amount found to be effective in treating the condition for which they are desired through relatively simple testing of differing concentrations and amounts of material. As one example, the inventive materials may be coated onto a tampon for insertion into the vaginal cavity for the pH control discussed at length above. Suitable tampons for the practice of this invention include those taught in US Patent 6,039,716 to Jessup et al., US Patent 5,795,346 to Achter et al. and US Patent 5,350,371 to Van Iten. The inventive materials may also be added to lotions, for example, for topical applications of treating agents like sunscreens.

The inventive materials may also be used as a delivery vehicle in capsule or tablet form for oral use when it is desired to treat conditions in the digestive tract. Such capsules may be designed using conventionally known methods and materials to remain intact in the mouth and throat and then dissolve in the stomach or intestine, thereby releasing the material of this invention where it is desired.

The inventive material may also be used advantageously in ophthalmic applications. The eye cleanses itself through blinking every few seconds, making topically applied treatments relatively short lived. A bioadhesive material could lengthen the residence time of such treatments if in the form, for example, of a solution, that could be placed on the inside of the eyelid or on the eye itself.

While many of the applications of this invention will be for use in human beings, the materials of this invention may also find wide application in the field of veterinary medicine. The delivery systems mentioned above may be particularly effective with animals since they often attempt to remove devices attached to their bodies. A bioadhesive, biodegradable polymer would clearly be advantageous in this application since it could be internally located and yet stationary, delivering the desired medicament to its target area for extended periods of time.

The two polymer components may be covalently bonded to each other forming graft, block or random copolymers. Such polymers are poly(acrylic acid)-graft-poly(lactic acid) (PAA-g-PLA) and poly(lactic-co-glycolic acid)-g-poly(acrylic acid) (PLGA-g-PAA), the making of which are described in the examples. The two components may also be incorporated into one material by forming a non-covalent interpolymer complex via hydrogen bonding. Typical examples in this category include the hydrogen bonding interpolymer complex formed between poly(methacrylic acid) and poly(lactic acid)-b-poly(ethylene glycol)-b-poly(lactic acid) tri-block copolymers.

### GENERAL OVERVIEW OF PROCESS

### GENERAL OVERVIEW OF PROCESS

More information on the making of functionalized biodegradable compositions may be found in US provisional patent application 60/389,026, attorney docket 17949, filed June 14", 2002 using express mail number EL 917864040 US, by the same inventors, and commonly assigned. This application teaches a new, simple and versatile method to functionalize biodegradable polymers by direct reaction of the biodegradable polymers in the medium of functional monomers, particularly vinyl monomers. An important point relevant to the current invention is that the commonly-used biodegradable polymers are soluble or dispersible in functional vinyl monomers.

In the practice of the 17949 invention generally, vinyl monomers (such as acrylic acid and HEMA) are polymerized in the presence of a biodegradable polymer (such as PLGA and PLA), with or without the presence of a solvent. The ratio of the monomer to biodegradable polymer may be from 10:90 to 90:10. More particularly, the ratio may be from 30:70 to 70:30 and still more particularly the ratio may be from 40:60 to 60:40.

The consideration on the choice of solvent is solubility of the reagents (monomer and biodegradable polymer) and possible interference with the polymerization process. For example, biodegradable polymers (such as PLA and PLGA) can be polymerized with vinyl monomers (such as acrylic acid and HEMA) in acetone. When the biodegradable polymer is soluble in the vinyl monomer, no solvent is needed for the latter to be polymerized in the presence of the former. Thus, both acrylic acid and HEMA were polymerized in the presence of PLA or PLGA without a solvent in the Examples below.

Polymerizations can be initiated by a free radical, ionic, cationic or an organometallic monomer. Depending on the nature of the initiator, polymerization may be initiated thermally or by radiation at elevated or room temperatures. An example of a free radical initiator useful for polymerizing vinyl monomers is 2, 2'-azobisisobutyronitrile (AIBN; 98%, from Aldrich Chem., catalog no. 44, 109-0, CAS 78-67-1). When AIBN is used as an initiator, heating the mixture to >50°C will initiate the polymerization. Typically, 0.01 - 2 weight percent of the initiator, relative to the monomer, may be used.

Alternatively, the monomers may be polymerized thermally or by radiation without the presence of an initiator.

The vinyl monomer may contain functional groups which may be chosen from carboxylic acid groups, hydroxyl groups, amine groups, chloride groups, sulfonic groups, phosphoric groups, aldehyde groups, oxirane groups, mercaptan groups, isocyanate groups, sulfide groups, activated amide groups, activated ester groups, and combinations thereof.

The biodegradable polymers may be chosen from aliphatic polyesters, copolymers of linear aliphatic polyesters and their copolymers, polyanhydrides, polyorthoesters, poly(ester-ether), polyamines, phosphorus-based polymers and combinations thereof. An aliphatic polyester may be chosen from polylactic acid, polyglycolic acid, poly(lactic-co-glycolic acid), their copolymers and combinations thereof.

The biodegradable polymer may be dissolved in the vinyl monomers. The method may also include a solvent.

Particularly notable combinations of biodegradable polymer and vinyl monomer include poly(lactic-co-glycolic acid) and acrylic acid.

Another aspect of the current invention concerns the combination of properties of these novel materials. The bioadhesive polymer components, particularly polycarboxylic acids, also have the properties of being hydrophilic, pH-responsive, and superabsorbent. Conversely, the biodegradable polymer components, particularly polyesters, have the properties of being hydrophobic and being latent pH modifiers through their degradation product acids. Novel materials having varied properties may result from the eclectic selection of starting materials.

As an example, it is possible to combine the pH-responsiveness of poly(carboxylic acid) and the latent pH-modifying property of biodegradable polyesters. It is also possible to make, for example, crosslinked gel particles, where either additional crosslinkers such as divinylbenzene are used or the hydrophobic aggregations of the biodegradable components themselves act as crosslinkers. Polycarboxylic acids are known pH-responsive polymers which adopt more expanded conformations at higher pH, swell the network and hence adsorb more water. On the other hand, more water will cause faster. degradation of the biodegradable polyesters and hence small molecular weight acids are released more rapidly, reduce the pH and help to relax the polycarboxylic acid chains to less expanded conformations. This can result in a self-regulated swelling/collapsing gel, which is highly desirable for controlled release systems.

As another example, the existence of the hydrophilic bioadhesive polymers may make the hydrophobic biodegradable polymers water soluble, swellable or dispersible. As also noted from the characterization results of some above samples, the copolymer may self-assemble into nano- or micro-particles in solution. It's believed that the particles will have the hydrophilic polymers at the surface and the hydrophobic polymers in the core section making biodegradable particles with bioadhesive surfaces, which is also highly desirable as drug delivery systems.

Another aspect of the current invention is, therefore, to include treating agents in the inventive composition and use them as drug delivery systems along with other biomedical applications. The treating agents useful herein are selected generally from the classes of medicinal agents, i.e., pharmaceutically active agents, and cosmetic agents. Substantially any agent of these two classes of materials that is a solid or liquid at ambient temperatures may be used in a composition of the present invention.

Exemplary medicinal agents include agents for treating cardiovascular conditions such as chlorothiazide (diuretic), propranolol (antihypertensive), hydralazine (peripheral vasodilator), isosorbide or nitroglycerin (coronary vasodilators), metoprolol (beta blocker), procainamide (antiarrythmic), clofibrate (cholesterol reducer) or coumadin (anticoagulant); agents for treating internal conditions such as conjugated estrogen (hormone), tolbutamide (antidiabetic), levothyroxine (thyroid conditions), propantheline (antispasmodic), cimetidine (antacid), phenyl propanolamine (antiobesity), atropine or diphenoxalate (antidiarrheal agents), docusate (laxative), or prochlorperazine (antinauseant); agents for treating mental health conditions such as haloperidol or chlorpromazine (tranquilizers), doxepin (psychostimulant), phenytoin (anticonvulsant), levo dopa (antiparkinism), benzodiazepine (antianxiety) or phenobarbital (sedative); anti-inflammatory agents such as fluorometholone, acetaminophen, phenacetin, aspirin, hydrocortisone, or predisone; anti-histamines such as diphenhydramine hydrochloride or dexchlorpheniramine maleate; antibiotics such as sulfanilamide, sulfamethizole, tetracycline hydrochloride, penicillin and its derivatives, cephalosporin derivatives or erythromycin; chemotherapeutic agents such as sulfathiazole, doxorubicin, cisplatin or nitrofurazone; topical anaesthetics such as benzocaine; cardiac tonics such as digitalis or digoxin; antitussives and expectorants such as codeine phosphate, dextromethorphan or isoproterenol hydrochloride; oral antiseptics such as chlor hexidine hydrochloride or hexylresorcinol; enzymes such as lysozyme hydrochloride or dextronase; birth control agents such as estrogen; opthalmic treating agents such as timolol or gentamycin. In addition, medicinal treating agents may also include insulin or interferon; polypeptide treating agents such as endorphins, human growth hormone, or still lower molecular weight polypeptides or conjugates of those polypeptides linked protein carriers. Exemplary cosmetic agents include sunscreens such as p-dimethylaminobenzoic acid or glyceryl p-aminobenzoate, a skin softener such as urea, keratolytic agents such as salicylic acid; acne treating agents such as benzoyl peroxide or sulfur; perfumes. Nutritional agents such as vitamins and/or minerals like riboflavin and iron, respectively, may also comprise useful treating agents herein. The treating agent may be used singly or as a mixture of two or more such agents.

One or more adjuvants may also be included with a treating agent, and when so used, an adjuvant is included in the meaning of the phrase "treating agent" as that phrase is used herein. Exemplary of useful adjuvants are chelating agents such as ethylenediaminetetracetic acid (EDTA) that bind calcium ions and assist in passage of medicinal agents through the mucosa and into the blood stream. Another illustrative group of adjuvants are the quaternary nitrogen-containing compounds such as benzalkonium chloride that also assist medicinal agents in passing through the mucosa and into the blood stream.

The treating agent is present in the compositions of this invention in an amount that is sufficient to prevent, cure and/or treat a condition for a desired period of time for which the composition of this invention is to be administered, and such an amount is referred to herein as "an effective amount". As is well known, particularly in the medicinal arts, effective amounts of medicinal agents vary with the particular agent employed, the condition being treated and the rate at which the composition containing the medicinal agent is eliminated from the body, as well as varying with the animal in which it is used, and the body weight of that animal. Consequently, effective amounts of treating agents may not be defined for each agent. Thus, an effective amount is that amount which in a composition of this invention provides a sufficient amount of the treating agent to provide the requisite activity of treating agent in or on the body of the treated animal for the desired period of time, and is typically less than that amount usually used.

The amounts of particular treating agents in the blood stream that are suitable for treating particular conditions are generally known, as are suitable amounts of treating agents used in cosmetics. It should be, therefore, a relatively easy task to formulate a series of controlled release compositions of this invention containing a range of such treating agents to determine the effective amount of such a treating agent for a particular composition of this invention. While the effective amount for all treating agents cannot be stated, typical compositions of this invention may contain one microgram to one gram of treating agent per dose administered.

The following are exemplary methods of producing the novel materials of this invention:

### Example 1: The making of macromonomer HEMA-PLA (Reference Example)

2-hydroxyethyl methacrylate (HEMA) from Aldrich Chemical Company of Milwaukee, Wisconsin, (catalog number 12863-5) was vacuum distilled at 67° C and 3.4 mmHg (453.3.3 Pa) to remove inhibitors. DL-lactide from Polysciences of Warrington, PA, (catalog number 16640) was recrystalized from toluene with a yield of 90.8 percent, and then vacuum dried at room temperature.

Lactide in the amount of 29.8 g (0.2 mol) was transferred to a rubber capped flask equipped with a magnetic stirrer, and 0.88 g stannous-2-ethyl hexanoate (0.3 weight percent catalyst/lactide) in 0.2 g toluene was added. The capped flask was connected to a vacuum for 4 hours before 1.3 g of HEMA (0.01 mol, 5 mol percent HEMA/lactide) was transferred to the flask by syringe. Nitrogen (N₂) was introduced to the flask via needle and the capped flask was immersed in a 130° C oil bath. The system was allowed to react for 4 hours with stirring. The mixture was then transferred to a beaker, allowed to cool to room temperature and put into a vacuum oven for 3 days at room temperature to remove any residual toluene.

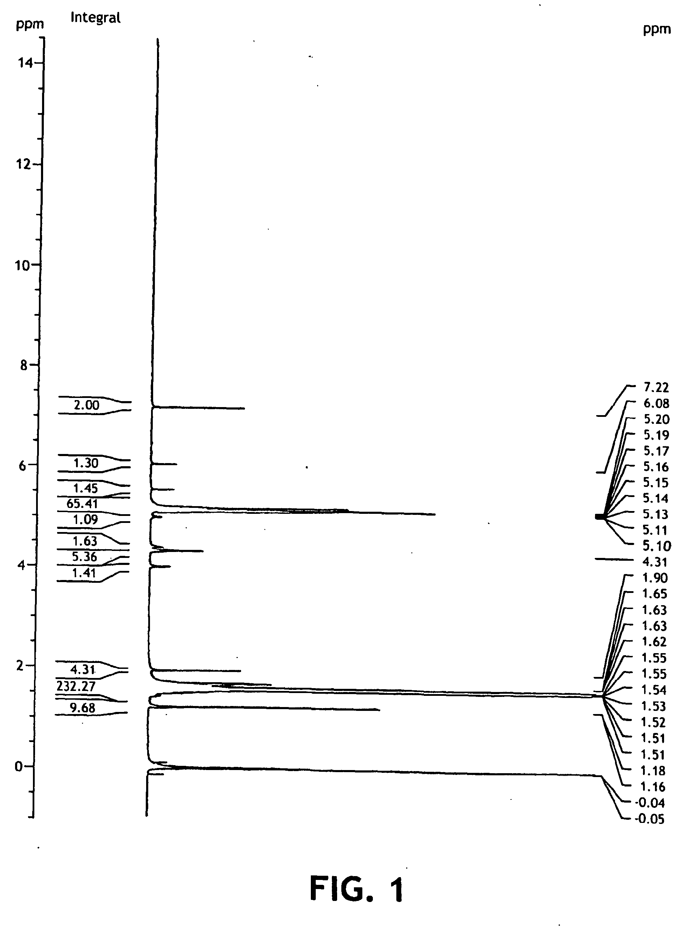

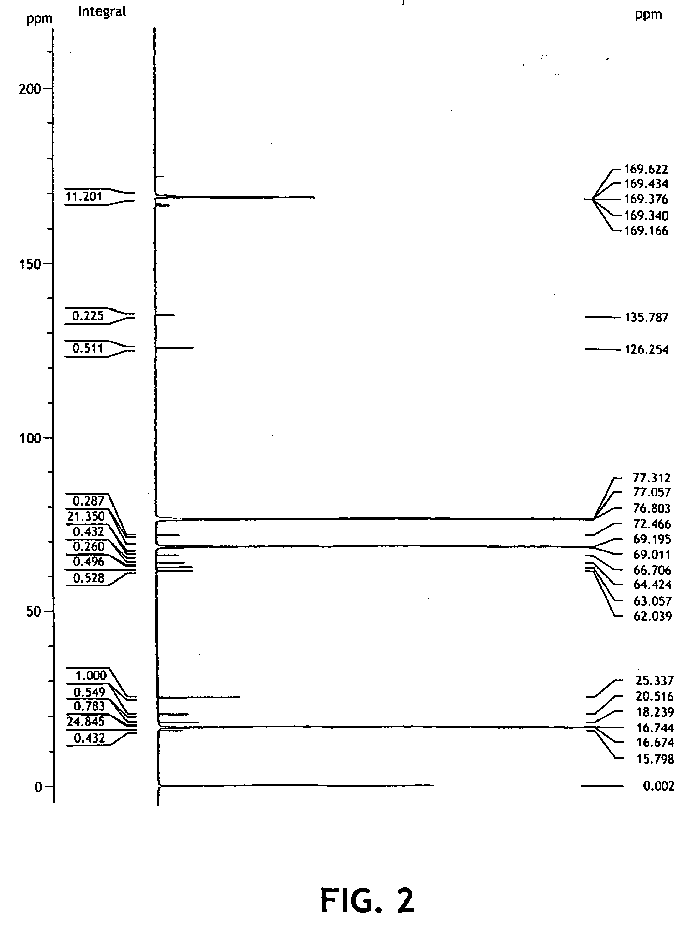

The obtained white powder was characterized by 1 H-and 13C-NMR (Figures 1 and 2). The peaks in 1 H-NMR spectrum were identified as the following: delta = 7.26 ppm (solvent H-chloroform impurity), delta - 6.12 and 5.6 (H-C=C), delta = 5.2 (methione proton in PLA blocks), delta = 4.4 (methione proton from the PLA-ending group), delta = 4.36 (methylene protons from HEMA part), delta = 1.94 (methyl protons from HEMA part), delta = 1.55-1.70 (methyl protons from PLA units), and delta =1.2 (water impurity). The peaks in 13 C-NMR spectrum were identified as the following: delta = 169 (C=O), delta = 126 and 135 (C=C), delta = 77 (CDCl3), delta = 69 (CH from PLA blocks), delta = 63 (CH2 from HEMA parts), and delta = 16.8 (CH3 from PLA blocks).

The functionality of the macromonomers (the average number of vinyl groups of each macromonomer) was determined from the 1 H-NMR peak intensity data of the methyl group from HEMA part (delta = 1.94, A=4.93) and the methione proton from the PLA ending groups (delta = 4.4, A=1.61), and the result was f =1.0. The molecular weight of HEMA-PLA macromonomers was calculated by using the 1H-NMR peak intensity data of the methione protons from PLA block units and the ending groups, and it was determined that the average number of lactic acid residues in each macromonomer was about 40.

### Example 2: PAA-g-PLGA Synthesis and Characterization

Acrylic acid from Aldrich, (catalog number 14723-0) was vacuum distilled to remove the inhibitors.

The polymerization mixture consisted of 5 g HEMA-PLA, 5 g acrylic acid, and 0.06 g 2, 2'-azobisisobutyronitrile (AIBN) 98% from Aldrich (cas 78-67-1, catalog no. 44, 109-0) (AIBN/AA=0.5/100 mol/mol) in 40 ml anhydrous DMF solvent. The reaction proceeded in an 80° C-oil bath, with stirring and N₂-bubbling for 24 hours.

After cooling to room temperature, the reaction mixture was poured into 800 mL of cold ether. The formed precipitate was allowed to settle down at the bottom, and then the upper layer of the clear solution was carefully removed. The precipitate was air-dried for 8 hours, before being transferred to a vacuum oven for 3 days at room temperature (22° C). The final polymer product weighted 7.1 g (yield 71%).

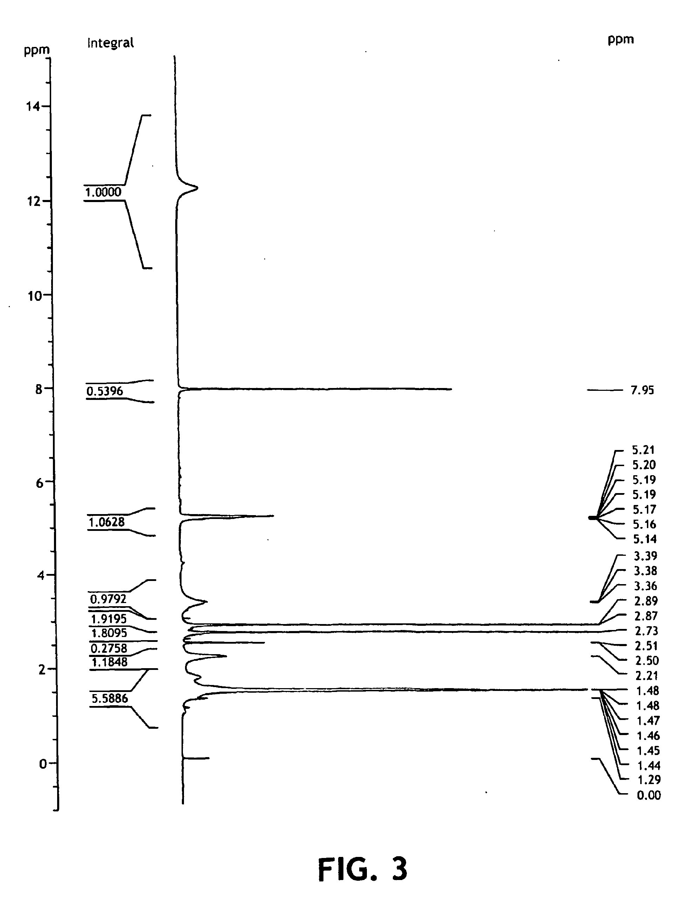

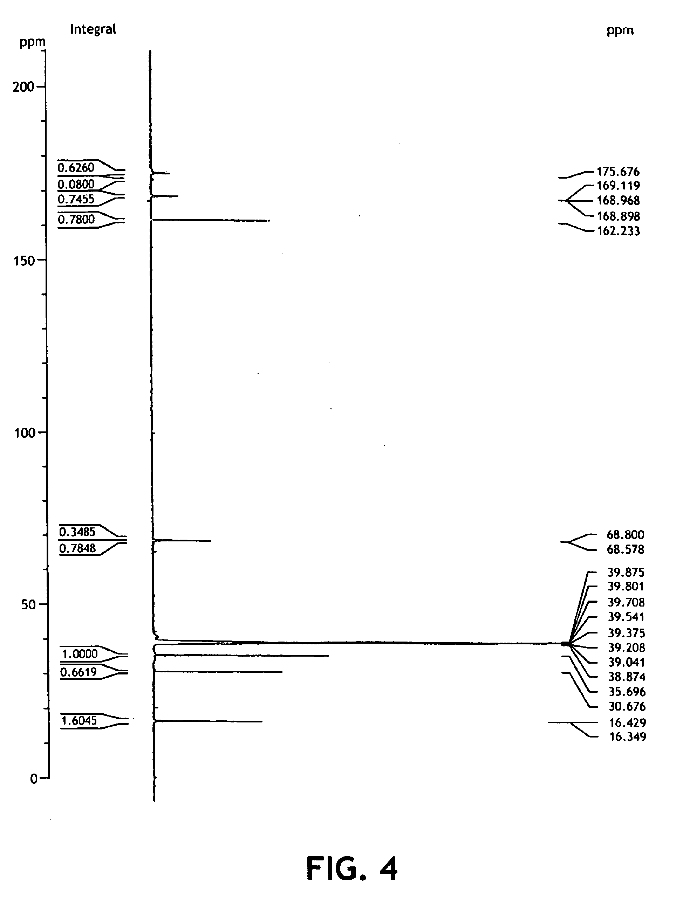

The product polymer was characterized by using 1H- and 13 C-NMR spectra, and the results are shown in Figures 3 and 4 with peak identification. One can see that the polymer contains both PLA and PAA groups.

Finally, the product polymer was tested for its solution structure by using a Coulter particle size analyzer. Three 0.1 weight percent PAA-g-PLGA solutions were made with different pHs: phosphate buffer saline (PBS) solution (pH 7.4), PBS+NaOH (pH 10.0), and NaOH solutions (pH 13). The polymer was dispersible in PBS solution and resulted in a milky solution, but the particles gradually precipitated in about one day. In contrast, the dispersion of PAA-g-PLGA in pH 10 and 13 solutions resulted in clear systems (average particles size 714 nm and 680 nm, respectively).

The difficulty of suspending PAA/PLGA copolymers in neutral pH conditions is presumably due to the PAA chains. It's believed that in aqueous solutions the PAA-g-PLGA polymer will self assemble into nano or micro particles with biodegradable cores and functional shell layers. It should also be noted that other properties can be added to the biodegradable polymers by modifying them with other monomers via the same methods.

### Example 3: PLGA-g-PAA Systems:

Two sets of experiments were done to prove firstly that PLGA is soluble in acrylic acid. (1) 1 g of PLGA (Medisorb® PLGA DL 5050 1A from Alkermes of Cambridge, MA) was mixed with 1 g acrylic acid (from Aldrich, Catalog no. 14723-0, vacuum distilled to remove inhibitors) and stirred for 40 minutes, the final mixture was homogeneous; and (2) 1 g PLGA was dissolved in 1g acetone. Then, 40 ml of acrylic acid was added to the solution, and resulted in a homogeneous and clear solution. The whole solution was poured into 800 mL water and white precipitation was observed.

As a test, 5.7 g PLGA was dissolved in 5.7 g acrylic acid with stirring. After the dissolution, 0.01 g AIBN (from Aldrich) was added into the solution, and the whole system was transferred into a 70° C oil bath. After 5 minutes, there was a self-acceleration effect observed and the whole system solidified. The mixture was put into a vacuum oven to remove the unreacted monomers.

The solution structure of the product polymer was also tested using a Coulter tester. The pH was adjusted with NaOH. In pH 9.6 solution, PLGA-g-PAA formed a milky solution with average particle size of 2 micron. The particle aggregation and precipitation became significant after one day of preparation. Further increasing pH to 13.7 resulted in a clear solution of PLGA-g-PAA with average particle size of 347 nm. The ¹H- and ¹³C-NMR spectra of the product polymer have been obtained. Both PLGA and PAA groups have been identified. Possible structures for PLGA-g-PAA are given in Figure 5 A, B and C.

### Example 5: interpolymer Complex between PMAA and PLA-PEG-PLA Polymers

Poly(lactic acid)-b-poly(ethylene glycol)-b-poly(lactic acid) tri-block copolymers PLA-b-PEG-PLA were first synthesized using the method of JL Hill-West et al., in Proceedings of National Academy of Science USA, 1994, 91, 5967-5971, with poly(ethylene glycol) (MW 2000, from Aldrich), DL-lactide (from Polysciences), and catalyst stannous-2-ethyl hexanoate (from ICN Biomedicals, Aurora, OH).

Poly(methacrylic acid) (PMAA) from Polysciences, in a solution of 5 weight percent was added dropwise to a 5 weight percent solution of the above made PLA-PEG-PLA in deionized water. White precipitate was formed. After being dried in a vacuum oven for 24 hours, a white material was obtained.

## Claims

1. A functionalized biodegradable polymer composition comprising a polycarboxylic acid bioadhesive polymer and a biodegradable polymer that have been incorporated into a single polymeric material by covalent bonding or by forming interpolymer complexes, selected from the group consisting of poly(acrylic acid)-graft-poly(lactic acid)(PAA-g-PLA), poly(lactic-co-glycolic acid)-g-poly(acrylic acid)(PLGA-g-PAA), a hydrogen bonding interpolymer complex formed between polycarboxylic acids and PEG-PLGA copolymers or PVA-PLGA copolymers, and a hydrogen bonded complex formed between poly(methacrylic acid) and poly(lactic acid)-b-poly(ethylene glycol)-b-poly(lactic acid) triblock copolymers.

2. The polymer composition of claim 1 which is in a form selected from the group consisting of solutions, gels, particles and liquid mixtures.

3. The polymer of claim 1 wherein said polycarboxylic acid is selected from the group consisting of poly(acrylic acid), poly(methacrylic acid), and their copolymers.

4. The polymer of claim 1 wherein said polycarboxylic acid contains about 80 percent of repeating units of carboxylic acid.

5. The polymer of claim 4 wherein said repeating units are selected from the group consisting of acrylic acid, methacrylic acid, fumaric acid, maleic acid, maleic anhydride, itaconic acid, crotonic acid and combination thereof.

6. The functionalized biodegradable polymer of claim 1 which is essentially free of a treatment agent.

7. The functionalized biodegradable polymer of claim 1 further comprising a treatment agent.

8. The polymer of claim 7 wherein said treatment is chosen from the group consisting of agents for treating menstruation disorders and infections, agents for treating cardiovascular conditions, agents for treating internal conditions, agents for treating mental health conditions, anti-inflammatory agents, chemotherapeutic agents, cardiac tonics, expectorants, oral antiseptics, enzymes, birth control agents, ophthalmic treating agents and combinations thereof.

9. A delivery vehicle comprising the polymer of any of claims 1 to 8.

10. The delivery vehicle of claim 9 wherein said vehicle is selected from the group consisting of solutions, cream, lotions, gels and vaginal inserts.

11. The vehicle of claim 10 which is a vaginal insert and said vaginal insert is selected from the group consisting of tampons, sponges, and rings.

12. A tampon comprising a functionalized biodegradable polymer according to any of claims 1 to 8.

13. A veterinary medicine delivery system comprising a functionalized biodegradable polymer according to any of claims 1 to 8.

14. The polymer composition according to any of claims 1 to 8, for use in maintaining or establishing a healthy body cavity environment.

15. The composition of claim 14, which is coated on a tampon.

16. The composition of claim 14, which is added to a lotion.

17. The composition of claim 14, which is added to a capsule or tablet.

## Patentansprüche

1. Eine funktionalisierte, biologisch abbaubare Polymerzusammensetzung, umfassend ein bioadhesives Polycarbonsäurepolymer und ein biologisch abbaubares Polymer, die in ein einzelnes Polymermaterial durch kovalente Bindung oder durch Formen von Interpolymerkomplexen eingebaut, ausgewählt aus der Gruppe bestehend aus Poly(Acrylsäure)- Graft- Poly(Milchsäure)(PAA-g-PLA), Poly(Milchsäure-co-Glykolsäure)-g-Poly(Acrylsäure)(PLGA-g-PAA), einem wasserstoffbindenden Interpolymerkomplex, der zwischen Polycarbonsäuren und PEG-PLGA Kopolymeren oder PVA-PLGA Kopolymeren gebildet wurde und einem wasserstoffgebundenen Komplex, der zwischen Poly(Methacrylsäure) und Poly(Milchsäure)-b-Poly(Ethylenglykol)-b-Poly(Milchsäure) Triblockcopolymeren gebildet wurde.

2. Die Polymerzusammensetzung nach Anspruch 1, welche in einer Form ist, die ausgewählt ist aus der Gruppe bestehend aus Lösungen, Gels, Partikel und flüssige Mischungen.

3. Das Polymer nach Anspruch 1, wobei das Polycarbonsäure ausgewählt ist aus der Gruppe bestehend aus Poly(Acrylsäure), Poly(Methacrylsäure) und deren Kopolymeren.

4. Das Polymer nach Anspruch 1, wobei die Polycarbonsäure ungefähr 80 Prozent sich wiederholende Einheiten von Carboxylsäure enthält.

5. Das Polymer nach Anspruch 4, wobei die sich wiederholenden Einheiten ausgewählt sind aus der Gruppe bestehend aus Acrylsäure, Methacrylsäure, Fumarsäure, Maleinsäure, Maleinsäureanhydrid, Itakonsäure, Crotonsäure und Kombinationen davon.

6. Das funktionalisierte, biologisch abbaubare Polymer nach Anspruch 1, welches im Wesentlichen frei ist von einem Behandlungsmittel.

7. Das funktionalisierte, biologisch abbaubare Polymer nach Anspruch 1, weiterhin umfassend ein Behandlungsmittel.

8. Das Polymer nach Anspruch 7, wobei die Behandlung ausgewählt ist aus der Gruppe bestehend aus Mitteln zur Behandlung von Menstruationsstörungen und Infekten, Mitteln zur Behandlung von kardiovaskulären Beschwerden, Mitteln zur Behandlung von inneren Beschwerden, Mitteln zur Behandlung von Beschweren betreffend die mentale Gesundheit, entzündungshemmenden Mitteln, chemotherapeutischen Mitteln, Herztoniken, Schleimlöser, orale Antiseptika, Enzymen, Mitteln zur Geburtenkontrolle, ophtalmischen Behandlungsmitteln und Kombinationen davon.

9. Ein Lieferträgerstoff umfassend das Polymer nach einem der Ansprüche 1 bis 8.

10. Der Lieferträgerstoff nach Anspruch 9, wobei der Trägerstoff ausgewählt ist aus der Gruppe bestehend aus Lösungen, Cremes, Lotionen, Gels und Vaginaleinsätzen.

11. Der Trägerstoff nach Anspruch 10, welcher ein Vaginaleinsatz ist und der Vaginaleinsatz ausgewählt ist aus der Gruppe bestehend aus Tampons, Schwämmen und Ringen.

12. Ein Tampon umfassend ein funktionalisiertes, biologisch abbaubares Polymer nach einem der Ansprüche 1 bis 8.

13. Ein Veterinärmedizinliefersystem, umfassend ein funktionalisiertes, biologisch abbaubares Polymer nach einem der Ansprüche 1 bis 8.

14. Die Polymerzusammensetzung nach einem der Ansprüche 1 bis 8 zur Verwendung beim Erhalten oder Herstellen einer gesunden Körperhöhlenumgebung.

15. Die Zusammensetzung nach Anspruch 14, welche auf einen Tampon beschichtet ist.

16. Die Zusammensatzung nach Anspruch 14, welche einer Lotion zugegeben ist.

17. Die Zusammensetzung nach Anspruch 14, welche einer Kapsel oder einer Tablette hinzugefügt ist.

## Revendications

1. Composition polymère biodégradable fonctionnalisée comprenant un polymère bioadhésif d'acide polycarboxylique et un polymère biodégradable qui ont été incorporés dans un matériau polymère unique par liaison covalente ou par formation de complexes interpolymères, choisis dans le groupe constitué par l'acide polyacrylique-greffe-acide polylactique (PAA-g-PLA), l'acide poly(lactique-co-glycolique)-g-acide polyacrylique (PLGA-g-PAA), un complexe interpolymère à liaisons hydrogènes formé entre des acides polycarboxyliques et des copolymères PEG-PLGA ou des copolymères PVA-PLGA, et un complexe à liaisons hydrogènes formé entre l'acide polyméthacrylique et des copolymères triséquencés acide polylactique-b-polyéthylène glycol-b-acide polylactique.

2. Composition polymère selon la revendication 1, qui est sous une forme choisie dans le groupe constitué par les solutions, les gels, les particules et les mélanges liquides.

3. Polymère selon la revendication 1, dans lequel ledit acide polycarboxylique est choisi dans le groupe constitué par l'acide polyacrylique, l'acide polyméthacrylique et leurs copolymères.

4. Polymère selon la revendication 1, dans lequel ledit acide polycarboxylique contient environ 80 pour cent d'unités de répétition d'acide carboxylique.

5. Polymère selon la revendication 4, dans lequel lesdites unités de répétition sont choisies dans le groupe constitué par l'acide acrylique, l'acide méthacrylique, l'acide fumarique, l'acide maléique, l'anhydride maléique, l'acide itaconique, l'acide crotonique et leurs combinaisons.

6. Polymère biodégradable fonctionnalisé selon la revendication 1, qui est essentiellement exempt d'un agent de traitement.

7. Polymère biodégradable fonctionnalisé selon la revendication 1, comprenant également un agent de traitement.

8. Polymère selon la revendication 7, dans lequel ledit traitement est choisi dans le groupe constitué par les agents pour le traitement des troubles de la menstruation et des infections, les agents pour le traitement des troubles cardiovasculaires, les agents pour le traitement des troubles internes, les agents pour le traitement des troubles de la santé mentale, les agents anti-inflammatoires, les agents chimiothérapeutiques, les toniques cardiaques, les expectorants, les antiseptiques oraux, les enzymes, les agents contraceptifs, les agents de traitement ophtalmiques et leurs combinaisons.

9. Véhicule de délivrance comprenant le polymère selon l'une quelconque des revendications 1 à 8.

10. Véhicule de délivrance selon la revendication 9, dans lequel ledit véhicule est choisi dans le groupe constitué par les solutions, les crèmes, les lotions, les gels et les inserts vaginaux.

11. Véhicule selon la revendication 10, qui est un insert vaginal et ledit insert vaginal est choisi dans le groupe constitué par les tampons, les éponges et les anneaux.

12. Tampon comprenant un polymère biodégradable fonctionnalisé selon l'une quelconque des revendications 1 à 8.

13. Système de délivrance d'un médicament vétérinaire comprenant un polymère biodégradable fonctionnalisé selon l'une quelconque des revendications 1 à 8.

14. Composition polymère selon l'une quelconque des revendications 1 à 8, pour une utilisation pour maintenir ou établir un environnement de cavité corporelle sain.

15. Composition selon la revendication 14, qui est revêtue sur un tampon.

16. Composition selon la revendication 14, qui est ajoutée à une lotion.

17. Composition selon la revendication 14, qui est ajoutée à une gélule ou un comprimé.
